# EUROPEAN PATENT APPLICATION

(11) **EP 2 055 772 A1**
(43) Date of publication of application: **06.05.2009**
(21) Application number: 07806111.6
(22) Date of filing: 27.08.2007
(51) Int. Cl.: C12N 5/06, A61L 27/00

(54) **METHOD FOR INHIBITING THE DEVELOPMENT OF TERATOMA**

(30) Priority: 28.08.2006 JP 2006230905
(71) Applicant: Matsumoto Dental University, Shiojiri-shi, Nagano 399-0781 (JP)
(72) Inventor: YAMAKI, Mariko, Shiojiri-Shi Nagano 399-0781 (JP); OZAWA, Hidehiro, Shiojiri-Shi Nagano 399-0781 (JP); EBINA, Satoshi, Shiojiri-shi, Nagano 3990781 (JP); ASASHIMA, Makoto, Tokyo 153-8902 (JP)
(74) Representative: Pautex Schneider, Nicole Véronique
(86) International application number: PCT/JP2007/066570
(87) International publication number: WO 2008/026548

(57) **Abstract**

The present invention provides a method for suppressing generation of a teratoma of an undifferentiated cell, preferably an ES cell. A biological material obtainable by the method and a therapeutic method for transplanting the biological material into a subject are also provided.

An undifferentiated cell, in particular, preferably an ES cell is cultured to produce an increased embryoid body-like and the embryoid body-like is cultured for at least one week on, and adhered to, a three-dimensional solid type-I collagen carrier or an analogue thereof. Furthermore, a biological material is obtained thereby and the biological material is transplanted into a mammalian subject.

## Description

### TECHNICAL FIELD

The present invention relates to a method for suppressing generation of a teratoma from an undifferentiated cell including an embryonic stem cell (as referred to an ES cell, below) in regenerative medicine. Furthermore, the present invention relates to a biological material obtainable by the method and also relates to a therapeutic method for transplanting the biological material into a subject.

### BACKGROUND ART

An ES cell is a cell that is pluripotential, has a high self-reproducibility and is capable of driving any cell, to which many scientists in the world have paid attention, as a material for regenerative medicine, capable of treating a disease as cannot have been treated in the current medicine. Furthermore, an existing ES cell line of a monkey or mouse is available and has been used for in studies (for example, see J.A. Thomson et al., Science 282, 1145 (1998)). For example, diabetes is caused by self-necrosis of a beta cell producing insulin in the pancreas, possibly due to its autoimmune reaction, and some level of recovery is made by a treatment of insulin injection however no fundamental therapeutic method has been found. Regenerative medicine for diabetes aims to derive a beta cell from an ES cell specifically and efficiently in vitro and transplant a purified beta cell into the body of a subject so that the subject is treated fundamentally (For example, see P. Blyszczuk, J. Czyz, G. Kania, M. Wagner, U. Roll, L. St-Onge, A. M. Wobus, Proc. Natl. Acad. Sci. U.S.A., 100, 998 (2003)., N. Lumelsky, O. Blondel, P. Laeng, I. Velasco, R. Ravin, R. McKay, Science 292, 1389 (2001), and B. Soria, E. Roche, G. Berna, T. Leon-Quinto, J.A. Reig, F. Martin, Diabetes 49, 157 (2000).)

Meanwhile, Parkinson's disease is caused by deficiency of a neurotransmitter named dopamine and there is a treatment of substitution therapy or the like but no fundamental therapeutic method has been found yet. However, regenerative medicine for Parkinson's disease aims to derive a nerve cell secreting dopamine from an ES cell specifically and efficiently in vitro and transplant a purified dopamine-producible cell into the body of a subject so that the subject is treated fundamentally (for example, see J. H. Kim, J. M. Auerbach, J. A. Rodriguez-Gómez, I. Velasco, D. Gavin, N. Lumelsky, S. H. Lee, J. Nguyen, R. Sanchez-Pernaute, K. Bankiewicz, R. McKay, Nature 418, 50 (2002)).

Thus, many scientists in the world have challenged in studies as to how a special cell needed for a subject or a precursor cell of the cell is produced from an ES cell specifically and efficiently in vitro.

However, some problems which are expected to occur after transplanting an intended cell into the body of a subject have remained in regenerative medicine using an ES cell. They are concerned about whether a transplanted cell sufficiently functions in the body of a subject, whether a blood vessel or a nerve forms a network and is fixed at a transplanted site, whether no teratoma (ES cell specific benign tumor) is produced, whether no immunological rejection occurs, and the like (for example, see Cookson, C. Sci. Amer. (July) A6-A11 (2005) and C.R. Freed, Proc. Natl. Acad. Sci. USA 99, 1755 (2002)).

Among these, it is very important to suppress generation of a teratoma. That is because it is considered that a teratoma generates from an "undifferentiated ES cell" included in a transplanted cell group. If there is a method capable of suppressing generation of a teratoma for a long period of time in the case where a cell group is transplanted into a subject, it is possible for an "undifferentiated ES cell" to be sufficiently differentiated into an intended cell in its body for the period of time, and accordingly, it is also expected that the transplanted cell group is fixed in the body of the subject due to blood vessels or neural networks and functions sufficiently so as to cure the subject of his/her disease. Furthermore, it is expected that immunological rejection can be solved by another technique, for example, deriving an intended cell from a custom-made ES cell in which the nucleus of somatic cells of a subject are transplanted, or the like (For example, see K. Hochedlinger, R. Jaenish, Nature 415, 1035 (2002), I. Wilmut, A. E. Shnieke, J. McWhir, A. J. Kind, K. H. Campbell, Nature 385, 810 (1997), D. Solter, Nature Rev. Genet. 1, 199 (2000), C. A. Cowan, J. Atienza, D. A. Melton, K. Eggan, K. Science 309, 1369 (2005), and Tada, Y. Takahama, K. Abe, N. Nakatsuji, T. Tada, Curr. Biol. 11, 1553 (2001)).

Thus, it is urgent and important to develop a method for suppressing generation of a teratoma in order to realize regenerative medicine utilizing an undifferentiated cell having multipotency, including an ES cell, but such a method has not been presented yet.

### DISCLOSURE OF THE INVENTION

### PROBLEMS TO BE SOLVED BY THE INVENTION

Therefore, the present invention has been made by taking the above-described matters into consideration, and aims to provide a method for suppressing generation of a teratoma after transplantation of an ES cell or an undifferentiated cell having multipotency. Furthermore, in regard to another object of the present invention, it aims to provide a biological material produced by the method. Moreover, in regard to yet another object of the present invention, it aims to provide a therapeutic method for transplanting the biological material into a subject in regenerative medicine.

### MEANS FOR SOLVING THE PROBLEM

The inventors had developed a method for forming a tooth using an undifferentiated cell prior to the present invention, and in a process of that invention, an odontoblast and ameloblast, which were materials constituting a tooth, could be derived from a mesenchymal cell originating from a tooth germ of a fetal mouse and a mosaic embryoid body-like of an ES cell of a mouse and it was observed that no tumorigenesis was caused after the mosaic embryoid body-like was transplanted into a mouse.

Herein, the inventors paid attention to this observation result, then have further studied actively, and as a result, and have found that no teratoma is generated from an ES cell in a body by culturing an ES cell or an embryoid body-like which is a mass of ES cells and adhered it to a three-dimensional solid type-I collagen carrier and making transplantation in the body of a mouse, thereby developing a unique method of the present invention.

That is, the above-described object is achieved by a method for suppressing generation of a teratoma **characterized in that** an undifferentiated cell is cultured to produce an increased embryoid body-like and the embryoid body-like is cultured on and adhered to a three-dimensional carrier, as described in claim 1. Thereby, an embryoid body-like increased by culturing an undifferentiated cell is sufficiently adhered to a three-dimensional carrier, and as a result, it is possible to suppress generation of a teratoma effectively.

The invention according to claim 2 is an invention as described in claim 1, **characterized in that** the undifferentiated cell is an ES cell. Thereby, an embryoid body-like increased by culturing an ES cell is sufficiently adhered to a three-dimensional carrier, and as a result, it is possible to suppress generation of a teratoma effectively.

The invention according to claim 3 is an invention as described in claim 1 or 2, **characterized in that** a time period for culturing the embryoid body-like on the three-dimensional carrier is at least one week. Thereby, an embryoid body-like increased by culturing an undifferentiated cell, in particular, an ES cell, is sufficiently adhered to a three-dimensional carrier, and as a result, it is possible to suppress generation of a teratoma effectively.

The invention according to claim 4 is an invention as described in any one of claims 1 to 3, **characterized in that** the three-dimensional carrier is made of natural type-I collagen or artificial type-I collagen analogous material or an analogue of the solid carrier. Thereby, an embryoid body-like increased by culturing an undifferentiated cell, in particular, an ES cell, is sufficiently adhered to a three-dimensional carrier which is a solid carrier of natural type-I collagen or artificial type-I collagen analogous material or an analogue of the solid carrier, and as a result, it is possible to suppress generation of a teratoma effectively.

The invention according to claim 5 is an invention as described in claim 4, **characterized in that** the three-dimensional carrier is coated with an ECM component. Thereby, an embryoid body-like increased by culturing an undifferentiated cell, in particular, an ES cell, is sufficiently adsorbed in a three-dimensional carrier coated with an ECM component, and as a result, it is possible to suppress generation of a teratoma effectively.

The invention according to claim 6 is an invention as described in claim 5, **characterized in that** the ECM component is one kind selected from the group including type-I collagens, laminins, and fibronectins. Thereby, an embryoid body-like increased by culturing an undifferentiated cell, in particular, an ES cell, is sufficiently adhered in a three-dimensional carrier coated with one kind selected from the group including type-I collagens, laminins, and fibronectins which are ECM components, and as a result, it is possible to suppress generation of a teratoma effectively.

The invention according to claim 7 is achieved by a biological material obtainable by a method as described in any one of claims 1 to 6. Thereby, it is possible to utilize it as a functional cell of, for example, a tissue, organ, which can be transplanted into a subject in regenerative medicine.

The invention according to claim 8 is achieved by a therapeutic method for a subject, **characterized in that** a biological material as described in claim 7 is transplanted into a subject. Thereby, it is possible to realize regenerative medicine capable of suppressing generation of a teratoma in a subject even if an ES cell is used.

### ADVANTAGEOUS EFFECT OF THE INVENTION

According to the present invention, it is possible to provide a method for suppressing generation of a teratoma, by producing an embryoid body-like increased by culturing an undifferentiated cell, in particular, preferably an ES cell and culturing the embryoid body-like on and adhering it to a three-dimensional solid type-I collagen carrier. According to the present invention, there is a possibility of providing an approach of regenerative medicine utilizing an intrinsic regenerative function of an organism, for example, culturing an undifferentiated cell regenerated in vitro in the body of an mammal and transplanting a biological material, for example, tissue or organ, of the present invention in which its embryoid body-like is cultured on and adhered to a three-dimensional solid carrier, so that a complete organ transplantation is performed without generation of a teratoma.

### BRIEF DESCRIPTION OF THE DRAWINGS

FIG. 1 is a diagram illustrating a protocol of the present invention.
FIG. 2 shows adhering of an embryoid body-like of ES cell to a three-dimensional type-I collagen carrier and transfer of the cell, wherein "A" and "B" are pictures showing a fluorescence microscope observation and a phase contrast microscope observation, respectively.
FIG. 3 is a picture showing generation of a teratoma.
FIG. 4 shows Hematoxilin-Eosin-staining images, wherein "A", "B", and "C" are pictures showing an embryoid body-like adhered to a collagen carrier to be transplanted, a negative control, and a positive control, respectively.
FIG. 5 shows specific staining of a blood vessel and a nerve, wherein the upper row and the lower row are pictures showing a vascular endothelial cell and a nerve cell, respectively.
FIG. 6 is a picture showing various kinds of cells or tissues regenerating in a teratoma.
FIG. 7 is a picture showing examination of teratoma generation with respect to various materials.
FIG. 8 is a picture showing examination of teratoma generation with respect to various kinds of ECM components.

### BEST MODE FOR CARRYING OUT THE INVENTION

One preferred embodiment of the present invention will be described in detail but the present invention is not limited to it.

The present invention is **characterized in that** a method for suppressing generation of a teratoma is derived by producing an embryoid body-like increased by culturing an undifferentiated cell capable of being differentiated into a variety of cells or tissues in a mammal and further culturing the produced embryoid body-like on and adhering it to a three-dimensional carrier.

First, an undifferentiated cell will be described which is referred to in the present invention. An undifferentiated cell refers to "an undifferentiated cell present in an embryo or a tissue of an organism", means a cell having a capability of making a cell identical to itself (self-replication capability) or a capability of differentiating into many kinds of cells constituting a tissue or organ (multipotency), and is also referred to as a stem cell (wherein undifferentiated cells and stem cells are generically referred to or described as undifferentiated cells, below). An undifferentiated cell is a cell having a capability of transforming, i.e. differentiating, into a particular cell when a signal of changing into a certain cell is received, and also having a capability of replicating or regenerating itself for a long term on its undifferentiated state before achieving its change. There is hierarchy in an undifferentiated cell wherein a more undifferentiated cell has a high self-replicating-capability and can be differentiated into very wide range of cell lines whereas a cell line into which an inferior undifferentiated cell is allowed to differentiate is limited.

An ES cell is generally called a universal cell and is an undifferentiated cell which is next high rank of a fertilized egg. In an organ of a mammal, a tissue-specific undifferentiated cell (that is, a somatic stem cell, also known as a tissue stem cell) is present. It is considered that a somatic stem cell has a capability of differentiating into a its specific line and a cell having the same capability as that of itself is regenerated on its division (self-replicated) thereby maintaining each tissue. That is, it is possible to extract an embryonic stem cell, i.e. ES cell, a somatic stem cell, and an embryo germ cell from an embryo, an adult, and a fetus, respectively.

In particular, an ES cell is known as a cell that self-replicates semipermanently while retaining its undifferentiated state under a certain culturing condition and has totipotency or multipotency whereby it is possible to differentiate into any kind of cells including a germ cell line.

An ES cell is isolated from a fertilized germ, that is, the initial stage of a germ under the condition that a fertilized egg is differentiated and evolving into a fetus, and is also called a multipotent stem cell because it has a capability of growing into any cell of a body. An ES cell has been studied for a "raw material" capable of creating each kind of cell which replaces a cell, tissue or organ whose function has been lost by an accident or disease, because it is isolated from an inner layer cell (inner cell mass) of a blastodermic vesicle at 5 or 6 days after its fertilization and cultured and it is possible to increase infinitely by means of culturing in a laboratory contrary to a somatic stem cell isolated from a human body while it has a universality such that it is possible to change to any cell. Theoretically, a method for transplanting an organ without causing a rejection is also considered in which a cell differentiated from an ES cell is recombined with an immunological gene by using a technique of gene therapy or a germ having genetic information of a subject is made and an ES cell is isolated therefrom and differentiated into an intended cell.

Meanwhile, a somatic stem cell refers to a cell at a state before its differentiation which is isolated from a tissue having been formed in a body. In a tissue, many cells which have particular functions in the tissue and have already been differentiated are present, and among these, an undifferentiated cell before that is differentiation into a cell having such a particular function is mixed. A somatic stem cell has a capability of replicating and producing a cell identical to itself and it is possible to create any individual cell in a tissue in which it is present, by means of its differentiation. Its representative example is a bone marrow stem cell or the like which has already been utilized for many therapeutics because it is known that it is differentiated into a particular tissue and which is used for bone marrow transplantation necessary for therapy of leukemia or the like.

Therefore, it is possible to achieve the present invention by using an undifferentiated cell having a function as described above.

Sampling and production of an undifferentiated cell used in the present invention are not particularly limited and may be conduced according to an arbitrary approach as described in publicly-known documents and the like. An undifferentiated cell used in the present invention is particularly preferably an ES cell of a mammal and may also be sampled and produced from, for example, a rodent (such as an ES cell of a mouse, an ES cell of a rat, or the like), a primate (such as an ES cell of a cynomolgus monkey, an ES cell of a human being, or the like), or the like, and such ES cells can be obtained from an embryonic germ cell of a mouse or human being. Furthermore, an ES cell may be obtained from a fertilized egg of a mammal by a method reported in a publicly-known document or the like or a commercially available ES cell may be used. For example, production of an ES cell of a mouse will be described which is a commonly used undifferentiated cell. A mouse is born at about 20 days after fertilization, and in its process, a shape of mouse is provided at about 12 days after generation. An ES cell originates from a state of blastodermic vesicle before beginning to provide its form, that is, at 3.5 days after generation of a mouse and after generation of a human being 1 week, respectively. In a blastodermic vesicle, there are two kinds of cells, wherein one is a rophectoderm that becomes a placenta and the other is an inner layer cell (inner cell mass) which produces every cell. Such an inner cell mass is isolated and cultured on a plate while a necessary chemical is added, thereby providing an ES cell. Such an ES cell can be increased if condition is right.

An ES cell is used in the present invention but no limitation to it is provided and it is possible to apply any of an undifferentiated cell of an organism to the present invention.

Next, a preferred method for culturing and increasing the above-mentioned undifferentiated cell to produce an embryoid body-like that is mass will also be described in detail in the under-mentioned specific example, wherein an ES cell is used as an undifferentiated cell and it is conducted according to the following approach by way of example.

An ES cell is cultured without a phalangeal cell (feeder cell) in a flask for culturing which is coated with 0.1% of gelatin, under the conditions of 37 °C and 5% of CO₂ for at least 5 days. In this case, replacement is commonly made every two days by using a culture solution, KnockOut DMEM containing 15% of knockout serum replacement (KSR), 0.1 mM of β-mercaptoethanol, 0.1 mM of a non-essential amino acid, 8 mM of L-glutamic acid, and 1000 U/ml of a leukemia inhibitory factor.

After culturing, increased cells are collected and treated with a trypsin EDTA solution to detach the cells and further pass through a filter with a diameter of 40 microns to provide a single cell. The solution containing thus treated cells is transferred to a low-binding 96-well plastic plate so as to each contain 1 × 10⁴ cells and cultured on an MSCGM medium (medium for a mesenchymal cell of a human being) at 37 °C and in 5% of CO₂ for 2 days to produce an embryoid body-like. Then, such an embryoid body-like is transferred to a low-binding 24-well plastic plate and further cultured on a similar medium and under the same condition as that described above for 3 days.

Then, the embryoid body-like is cultured, together with a three-dimensional carrier, on an MSCGM medium at 37 °C and in 5% of CO₂ and adhered to the three-dimensional carrier. The embryoid body-like produced by the above-mentioned method is cultured on, for example, a three-dimensional carrier with 1 - 2 × 1 - 2 × 1 - 2 mm and culturing on a similar medium and under the same condition as that described above is conducted for at least one week. In this process, the embryoid body-like is adhered to the three-dimensional carrier.

Furthermore, a three-dimensional carrier used in the present invention is preferably a solid three-dimensional solid type-I collagen carrier. A type-I collagen is one kind of extraxellular matrix (ECM) component and fills between cells. For major components, there are provided, type-I collagens, type-IV collagens, laminins, fibronectins, and the like. Herein, it is possible to use a commercially available three-dimensional type-I collagen carrier and a type-I collagen used in the present invention may be a natural one or an artificially-produced similar material.

Moreover, another analogue having a similar effect may be used for a three-dimensional carrier used in the present invention as far as it is possible to culture and adhere the above-mentioned embryoid body-like, and in this case, the three-dimensional carrier is coated with an ECM component. Coating of the three-dimensional carrier with an ECM component is conducted by dipping the three-dimensional carrier in an ECM solution at 37 °C and in 5% of CO₂ overnight and subsequently drying it in a safety cabinet for one day. Particularly, preferred ECM components in the present invention are type-I collagens, laminins, and fibronectins.

Therefore, in the present invention, it is possible for an embryoid body-like increased by culturing an undifferentiated cell to be cultured on and adhered to a three-dimensional carrier more sufficiently, on the condition that one kind of ECM component which is a type-I collagen, a laminin, or a fibronectin is applied on the three-dimensional carrier, independently of the material of the three-dimensional carrier. Thereby, it is possible to suppress a capability of forming a teratoma, because an undifferentiated cell adhered to a three-dimensional carrier is moderately differentiated on the three-dimensional carrier. Accordingly, it can be understood that the presence of a type-I collagen, a laminin, or a fibronectin is required in order to suppress generation of a teratoma effectively.

Thus, an embryoid body-like increased by culturing an undifferentiated cell is further cultured on and adhered to a three-dimensional carrier, thereby providing a biological material, and it is possible to provide the biological material as a functional cell that is allowed to be transplanted to a desired affected part of a subject together with the adhering three-dimensional carrier. That is, it is possible to repair a damage of a tissue, organ or the like which is caused by a disease, accident or the like by using a biological material according to the present invention. In other words, a biological material according to the present invention is effectively available for so-called regenerative medicine and a therapeutic method for transplanting the above-mentioned biological material into an affected part of a subject is provided. Herein, it is possible to transplant the above-mentioned biological material into a desired affected part of a mammalian subject by means of a commonly used and well-known transplantation surgery, for example, a surgical operation such as infusion graft. Furthermore, it is possible to avoid a problem of immunological rejection by utilizing a somatic stem cell of a subject himself/herself as an undifferentiated cell. Moreover, with respect to a subject, it is possible to apply it to a general mammal including a human being.

In addition, an embryoid body-like increased by culturing an undifferentiated cell is adhered to a three-dimensional carrier by a therapeutic method for transplanting the above-mentioned biological material into an affected part of a subject in the present invention whereby (1) it is possible to keep a sufficient number of cells acting on a local portion of an affected part and (2) it is possible to conduct infusion graft of a biological material (as an intended cell) and subsequently fix it on the affected part, so as to be used as an effective therapy of regenerative medicine.

Furthermore, in regard to an advantage of using a three-dimensional carrier in the present invention, it is necessary or essential to provide an intended three-dimensional cell as well as an "transplantation of a two-dimensional cell" which has been studied until now, when repair of a great damage of a tissue, which is caused by a disease, accident or the like, regeneration of organ, or the like is required, and a three-dimensional carrier of ECM component which is harmless for an organism and is absorbed after its transplantation is one of very useful transplantation means.

Therefore, according to the present invention, a regenerative medicine utilizing an undifferentiated cell such as an ES cell is realized without generating a teratoma by producing an embryoid body-like increased by culturing an undifferentiated cell, culturing the embryoid body-like on and adhering it to a three-dimensional carrier, and transplanting a biological material obtained thereby into an affected part of a mammalian subject.

### SPECIFIC EXAMPLES

Specific examples implemented in accordance with a method of the present invention will be described in more detail below but the present invention is not limited to these specific examples. Additionally, various embodiments are available for their details without departing from the spirit and scope of the present invention.

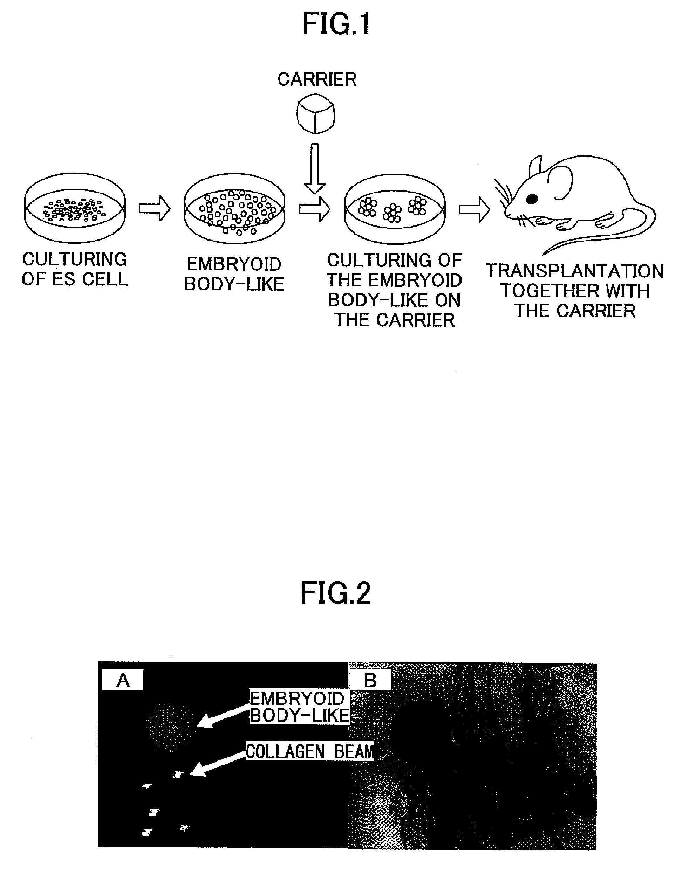

In the present specific example, ES cells were cultured and increased and embryoid body-like that was masses of those ES cells were produced, as shown in FIG. 1. Furthermore, the produced embryoid body-like was cultured for at least one week together with, and adhered to, a three-dimensional solid type-I collagen carrier. Then, the embryoid body-like which was thus cultured on and adhered to the solid three-dimensional carrier was transplanted under renal fascia of an immunodeficient mouse together with the three-dimensional carrier. After the transplantation, observation was conducted for 7 weeks as to whether a teratoma was created or not.

The above-mentioned present specific example will be described in more detail below.
- Method for culturing undifferentiated cells -

In the present specific example, ES cells (from a mouse) were used among the above-mentioned undifferentiated cells.

The ES cells produced in accordance with a paper (home-made one to which a GFP was adhered, K. Eggan, H. Akutsu, L. Loring, L. Jackson-Grusby, M. Klemm, WM III, Rideout, R. Yanagimachi, R. Jaenisch, Proc. Natl. Acad. Sci. U.S.A. 98, 6209 (2001)) was used.

These ES cells were seeded onto a flask for culturing which was coated with 0.1 % of gelatin (Cosmo Bio), without a phalangeal cell (feeder cell), and cultured under the conditions of 37 °C and 5% of CO₂ for 5 days. Its culture solution is KnockOut DMEM (Gibco) which contained 15% of a knockout serum replacement (KSR), 0.1 mM of β-mercaptoethanol (Cosmo Bio), 0.1 mM of a non-essential amino acid (Cosmo Bio), 8 mM of L-glutamic acid (Cosmo Bio), and 1000 U/ml of a leukemia inhibitory factor (Chemicon). The culture solution was normally replaced every 2 days.

After the culturing, increased ES cells were collected, treated with a trypsin EDTA solution (Gibco) to detach the cells, and further passed through a filter with a diameter of 40 microns (Falcon) to provide a single cell. The solution containing thus treated ES cells was transferred to a low-binding 96-well plastic plate (Nunc) so as to each contain 1 × 10⁴ ES cells and cultured at 37 °C and in 5% of CO₂ for 2 days to produce an embryoid body-like. Then, the produced embryoid body-like was transferred to a low-binding 24-well plastic plate (Nunc) and further cultured on the same medium and under the same condition for 3 days. When culturing for 1 day after seeding the low-binding 96-well plastic plate, the cells rounded, but because these cells had still been small and the number of the cells was insufficient, they were transferred to a low-binding 24-well plastic plate (that is, a serum medium) so as to feed a nutrient sufficiently and grow them.

Then, the embryoid body-like was cultured, together with a three-dimensional type-I collagen carrier with 1 - 2 × 1 - 2 × 1 - 2 mm (commercial name: Collagen Sponge Honeycomb^{™}, KOKEN CO., LTD.), on an MSCGM medium at 37 °C and in 5% of CO₂ for 1 week and adhered to it (FIG. 2). It was found that the embryoid body-like adhering to the three-dimensional type-I collagen carrier adhered to and extended on a beam portion of the collagen carrier with a honeycomb structure.

### - adhering of cells to a three-dimensional type-I collagen carrier and their movement -

The embryoid body-like started to break and their cells moved along the beam of the carrier immediately after the embryoid body-like was cultured together with the three-dimensional type-I collagen carrier. As observation was made by a microscope at one week after the culturing, sufficient adhering could be confirmed.

### - Observation of teratomas -

The embryoid body-like boding to the three-dimensional type-I collagen carrier was transplanted under the left-renal fascia of an immunodeficient mouse (commercially available SCID mouse) together with the three-dimensional carrier (sample group). No transplantation was applied under the right-renal fascia of the same mouse (negative control group). Furthermore, for a positive control group, it was confirmed that the embryoid body-like which did not adhere to a three-dimensional type-I collagen carrier was similarly transplanted under the left-renal fascia of a mouse and a teratoma was necessarily generated. Additionally, the method of transplantation for a mouse was infusion graft conducted according to a conventional method. Observation was made for 7 weeks after the transplantation and the observation was made as to whether a teratoma was generated or not.

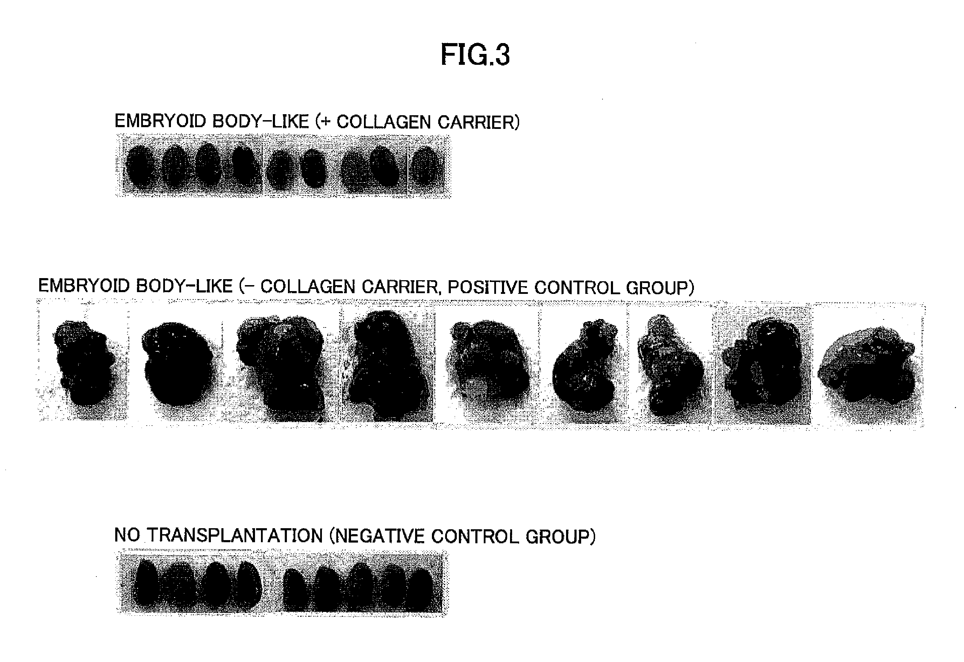

From the result of the observation, the embryoid body-like binding to the three-dimensional type-I collagen carrier ultimately generated no teratoma for all of nine cases (the upper figure of FIG. 3).

Thus, generation of a teratoma was suppressed well by sufficiently adhering an embryoid body-like to a three-dimensional type-I collagen carrier for at least one week. That is, it could be found that it was possible to suppress generation of a teratoma completely by sufficiently adhering an embryoid body-like to a three-dimensional type-I collagen carrier. Furthermore, in the example of the upper figure of FIG. 3 in which no teratoma was generated, no new generation of a teratoma was found even when 27 weeks had passed after transplantation. On the other hand, all of the embryoid body-like which did not adhere to a three-dimensional type-I collagen carrier (positive control group) generated a teratoma until 7 weeks after transplantation (the middle figure of FIG. 3). Furthermore, no generation of a teratoma was found in the negative control group in which no embryoid body-like was transplanted (the lower figure of FIG. 3). A similar test was also conducted for a cell solution of ES cells before creating an embryoid body-like and the result that was completely identical to that of the embryoid body-like was obtained. Many studies have reported that a teratoma is usually formed at about 3 - 4 weeks after only ES cells are transplanted into a mouse. From our experiments, it was found that, when keeping was made continuously, death was caused one after another due to a teratoma and almost complete annihilation was caused during 7 weeks. From the above-mentioned specific examples, it is found that it is possible to suppress generation of a teratoma completely if a time period for culturing an embryoid body-like increased by culturing an ES cell and a three-dimensional type-I collagen carrier is at least one week and its sufficient adhering is confirmed by a microscope.

### - Images of transplantation tissues -

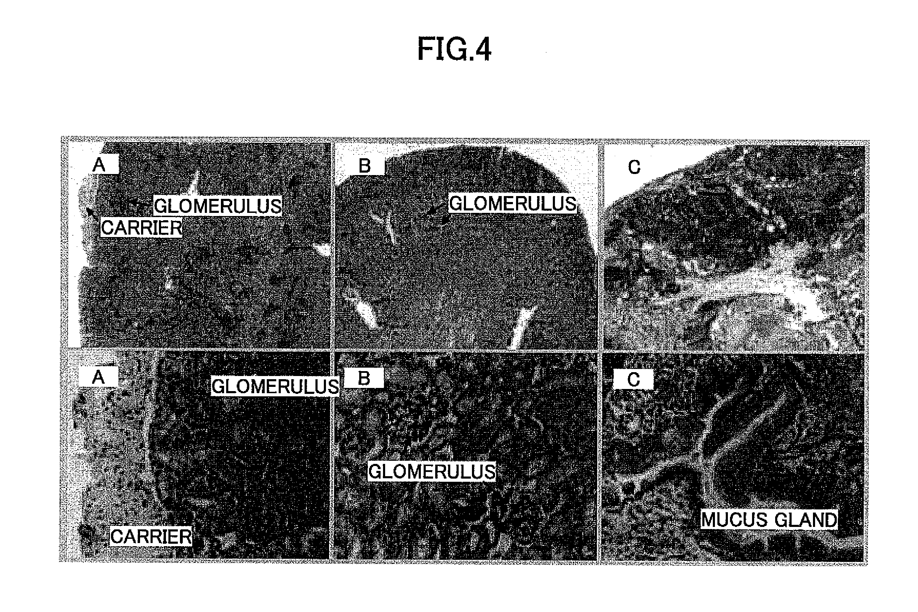

The transplanted embyoid body-like was detached together with a kidney and fixed by 4% of paraformaldehyde, embedded in paraffin, sliced, and hematoxilin-eosin-stained and tissue images were observed (FIG. 4). There was no abnormal finding in the kidney in which the embryoid body-like was adhered to the three-dimensional type-I collagen carrier and transplanted (FIG.-4A), similarly to that of the negative control group (no transplantation, FIG. 4B). On the other hand, in the positive control group (embryoid body-like adhering to a collagen carrier, FIG. 4C), a teratoma was formed in a kidney and a tridermic structure such as a muciparous gland, cartilage, muscle, nerve, or blood vessel was formed.

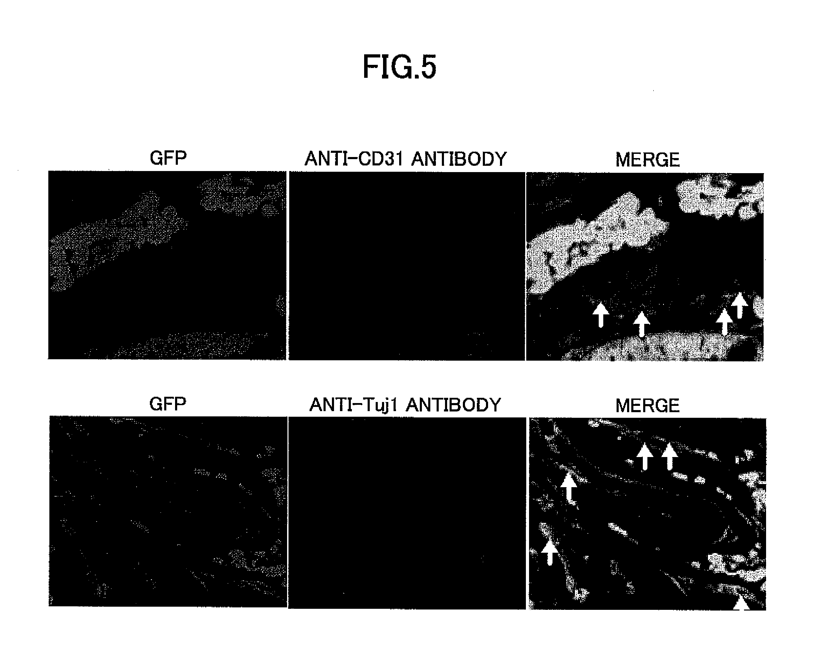

Then, the embryoid body-like adhering to the three-dimensional type-I collagen carrier and transplanted were immunofluorescence-stained with an anti-CD31 antibody (PECAM, R&D Systems) (the upper figure of FIG. 5) and an anti-Tuj1 antibody (Convance) (the under figure of FIG. 5) and the development of a vascular endothelial cell and a nerve cell was observed. Because GFP was green and antibody-staining was red (rhodamine), an overlapping portion of them was yellow. This indicated that a cell derived from an ES cell and expressed GFP was stained with an antibody. In addition, a nucleus was immunofluorescence-stained with DAPI (Molecular Probe) (blue). Because CD31 was specifically expressed for a vascular endothelial cell, a red stained cell indicated a vascular cell.

Meanwhile, because Tuj1 stained a nerve-specific β-tubulin, a red stained cell indicated a nerve cell. Both the vascular endothelial cell and the nerve cell overlapped with GFP fluorescence (green), overlaps were yellow. That is, it was confirmed that both a vascular cell and a nerve cell were derived from ES cells (see FIG. 5).

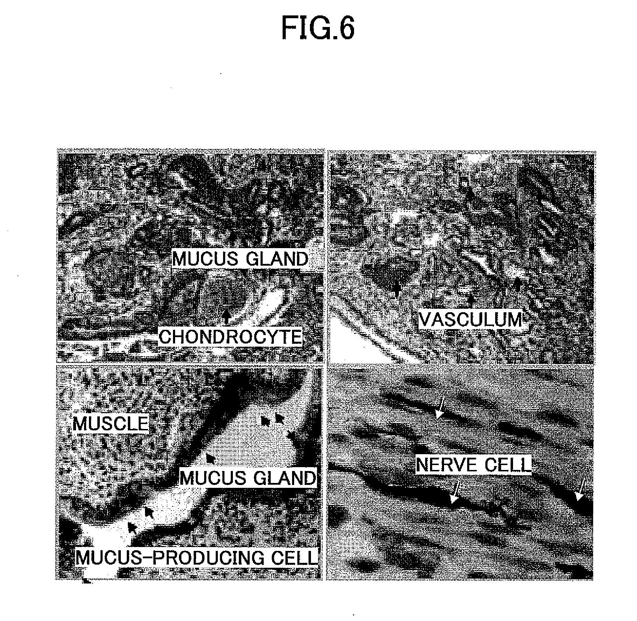

Then, the detached teratoma kidney (FIG. 4C) was fixed and embedded in paraffin as described above, immunofluorescence-stained with an anti-GFP antibody, and color-developed (brown) by a peroxidase method using DAB, and a cell nucleus was further stained with hematoxilin. From these double staining images, it was found that various kinds of cells or tissues, such as a cartilage cell, a nerve cell, a mucilage-producing cell, a mucous gland, a muscle, and a luminal structure were created in a teratoma kidney, wherein they were tridermic and were derived from ES cells (see FIG. 6).

### - Effect of another carrier -

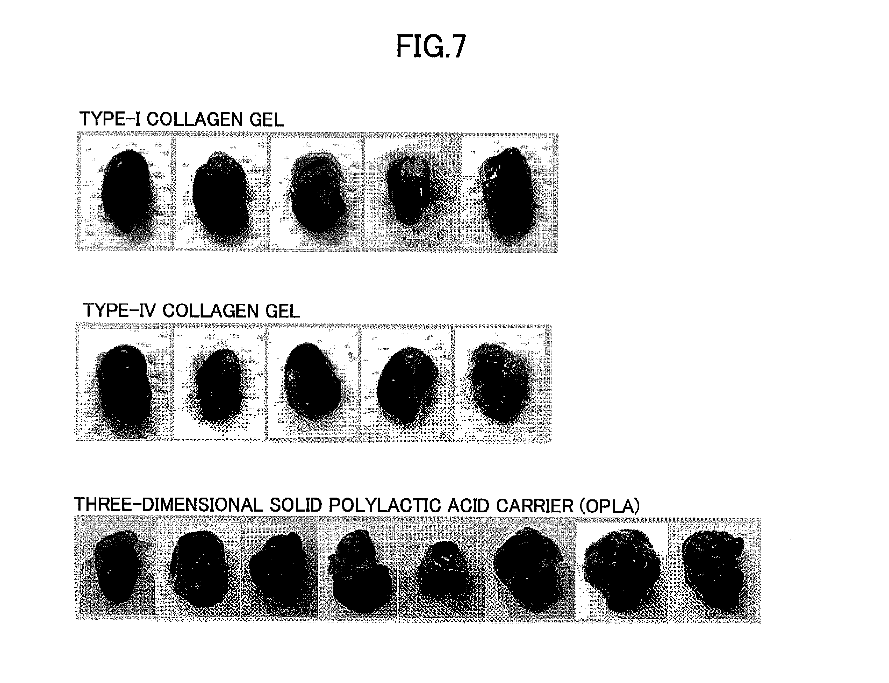

As described above, generation of a teratoma from ES cells could be suppressed efficiently by adhering embryoid body-like increased by culturing ES cells to a solid three-dimensional type-I collagen carrier. Furthermore, in order to confirm whether such a teratoma-suppressing effect is a specific effect of the type-I collagen and whether another solid carrier has a similar suppression effect, the same tests as that of FIG. 1 were conducted for a type-I collagen gel (Nitta Gelatin Inc.), a gel made of a type-IV collagen (BD Biosciences), and a three-dimensional solid polylactic acid carrier (BD Biosciences; OPLA) according to a method similar to the above-mentioned one. Their results are shown in FIG. 7. Additionally, polylactic acid is an artificial product, and however, has been used for transplantation as being harmless, because it is decomposed in vivo. Furthermore, "polylactic acid" is a produce produced at a time when an organism creates energy. As seen from FIG. 7, a teratoma-suppressing effect was apparently found in a gel-like carrier except a solid type-I collagen carrier, but the gel was rapidly digested in a body whereby there was a possibility of disappearing of the suppressing effect, and tertomas were formed ultimately.

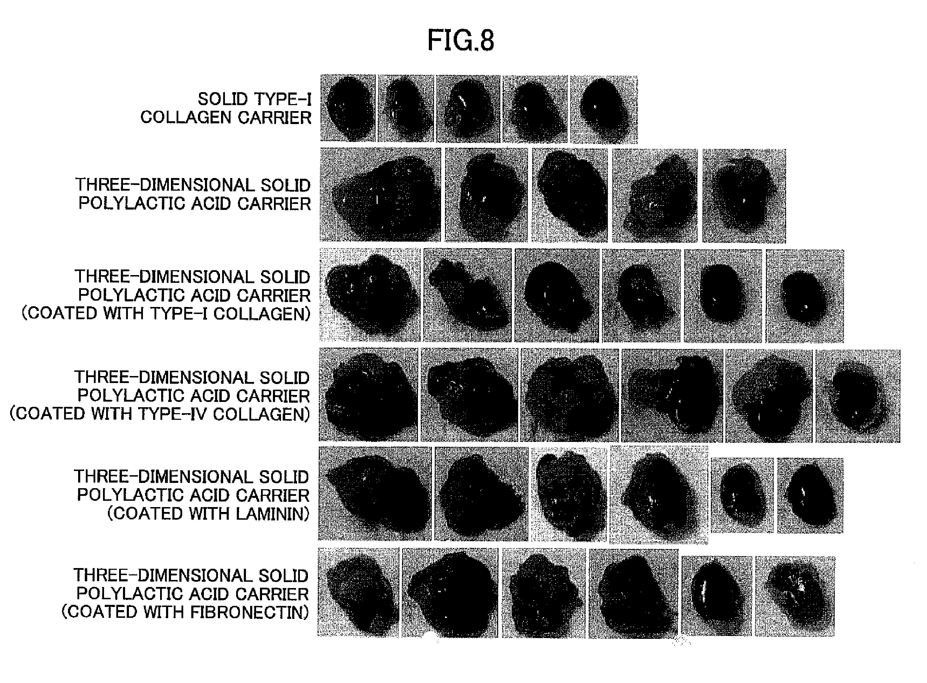

Moreover, three-dimensional carriers in which a type-I collagen (Nitta Gelatin Inc.), a type-IV collagen (Nitta Gelatin Inc.), a laminin (Gibco), and a fibronectin (Gibco) were applied to the three-dimensional polylactic acid carrier did not control teratoma formation at all (see FIG. 7) were produced for the purpose of investigating a teratoma-formation-capability-controlling effect of an ECM component other than the above-mentioned type-I collagen. The application was conducted by dipping the three-dimensional carrier in each ECM solution at 37 °C and in 5% of CO₂ overnight and subsequently conducting its drying in a safety cabinet for 1 day. A series of experiments for adhering embryoid body-like increased by culturing ES cells similar to those described above to each three-dimensional carrier described above and transplanting them was conducted in accordance with a method similar to that described above and observation was made with respect to the presence or absence of a tertoma at 7 weeks after the transplantation (see FIG. 8).

As a result, although the control three-dimensional type-I collagen carrier most strongly controlled its teratoma formation capability, a significant control effect was also found in each of the three-dimensional carriers coated with the laminin and the fibronectin. Because the control rates of the type-I collagen and each of the laiminin and fibronectin were 50% and 33%, respectively, even under the condition that teratoma formation was poorly controlled, i.e., their application was made on the three-dimensional carrier of polylactic acid which did not control teratoma formation at all, it is understood that laminins and fibronectins have a teratoma-controlling effect.

**Table 1**

| Sample | 3-D solid Type-I collagen carrier | 3-D solid PolyLA carrier | 3-D solid PolyLA carrier (type-I collagen coating) | 3-D solid PolyLA carrier (type-IV collagen coating) | 3-D solid PolyLA carrier (laminin coating) | 3-D solid PolyLA carrier (fibronectin coating) |
|---|---|---|---|---|---|---|
| Control (rate) | 5/5 (100%) | 0/5 (0%) | 3/6 (50%) | 0/6 (0%) | 2/6 (33%) | 2/6 (33%) |

| | | | | | | |
|---|---|---|---|---|---|---|
| 3-D = three-dimensinal PolyLA = Polylactic acid | | | | | | |

Therefore, according to the present invention, an increased embryoid body-like is produced by culturing an undifferentiated cell and the embryoid body-like is cultured on and adhered to a three-dimensional carrier, whereby it is possible to suppress generation of a teratoma of ES cell, and a biological material obtained thereby is transplanted into a desired affected part of a subject, whereby it is possible to realize a regenerative medicine utilizing an undifferentiated cell including an ES cell without generation of a teratoma.

Although a preferred specific example of the present invention has been described above, the present invention is not limited to such a specific embodiment and various alterations or modifications are allowed within the scope of the present invention as described in the claims.

### INDUSTRIAL APPLICABILITY

Because, according to the present invention, it is possible to solve a problem of generation of a teratoma after transplantation which problem is a drawback possessed by an ES cell expected to hold the key of regenerative medicine, considerable medical effect such that studies of regenerative medicine drastically progress and a part or lot of regenerative medicine is realized is expected when the present method or its modified technique is known in the world. This is effective for the QOL (quality of life) of a subject in person, and in addition, it is expected to greatly contribute to reduction of increasing medical expenses. Furthermore, it is also expected to be effective for a test system of suppressing generation of a teratoma in order to elucidate the characteristics of an undifferentiated cell including an ES cell and develop a safe method for regenerative medicine. Thus, the present invention opens up all the possibilities described above and the prospect of its practical realization and its utility value in industry are extremely high.

The present international application claims its priority based on Japanese Patent Application No. 2006-230905 filed on August 28, 2008, the entire content of which is incorporated by reference for the present international application.

## Claims

1. A method for suppressing generation of a teratoma, **characterized by** culturing an undifferentiated cell to produce an increased embryoid body-like and culturing the embryoid body-like on and adhering it to a three-dimensional carrier.

2. The method according to claim 1, **characterized in that** the undifferentiated cell is an ES cell.

3. The method according to claim 1 or 2, **characterized in that** a time period for culturing the embryoid body-like on the three-dimensional carrier is at least one week.

4. The method according to any one of claims 1 to 3, **characterized in that** the three-dimensional carrier is a solid carrier of natural type-I collagen or artificial type-I collagen analogous material or an analogue of the solid carrier.

5. The method according to claim 4, **characterized in that** the three-dimensional carrier is coated with an ECM component.

6. The method according to claim 5, **characterized in that** the ECM component is one kind selected from the group consisting of type-I collagens, laminins, and fibronectins.

7. A biological material obtainable by the method according to any one of claims 1 to 6.

8. A therapeutic method for a subject, **characterized by** transplanting the biological material according to claim 7 into a subject.
